# EUROPEAN PATENT SPECIFICATION

(11) **EP 3 087 727 B1**
(45) Date of publication and mention of the grant of the patent: **05.06.2019**
(21) Application number: 13824517.0
(22) Date of filing: 24.12.2013
(51) Int. Cl.: A61B 5/00, A61B 5/16, G06K 9/00, G06K 9/62, H04N 5/232

(54) **AN EMOTION BASED SELF-PORTRAIT MECHANISM**
GEFÜHLSBASIERTER SELBSTPORTRÄTMECHANISMUS
DISPOSITIF D'AUTOPORTRAIT BASE SUR LES EMOTIONS

(43) Date of publication of application: 02.11.2016
(73) Proprietor: Sony Corporation, Tokyo 108-0075 (JP)
(72) Inventor: THÖRN, Ola, S-216 12 Limhamn (SE)
(74) Representative: Aera A/S
(86) International application number: PCT/IB2013/002863
(87) International publication number: WO 2015/097487

(56) References cited:
- US-A1- 2007 201 725
- US-A1- 2008 240 518

## Description

### TECHNICAL FIELD OF THE INVENTION

A disclosed implementation generally relates to causing an imaging device, such as a camera or a video device, to selectively capture images and more particularly to predict emotional events and to selectively capture images associated with the emotional events.

### DESCRIPTION OF RELATED ART

Many different types of devices are available today for taking pictures, editing the captured images, and distributing the images. For example, a user may take a picture using a smart phone, a camera, or an augmented reality device. The user may take and share self-portraits and/or images of surroundings. For example, the user may cause images to be captured at regular intervals (e.g., hourly). However, the user may not wish to sift through and delete numerous, uninteresting images (such as images of the user sleeping) to find a small number of interesting images. US 2007/201725 A1 describes an unsatisfactory scene is disqualified as an image acquisition control for a camera. An image is acquired. One or more mouth regions are determined. The mouth regions are analyzed to determine whether they are frowning, and if so, then the scene is disqualified as a candidate for a processed, permanent image while the mouth is completing the frowning. US 2008/240518 A1 describes an image capture apparatus comprises a first imaging device operable to capture an image feedback image, generator for generating a feedback image from a captured image for output to a display, a facial recognition unit operable to determine the position of a face within the feedback image, an image corruptor operable to corrupt the feedback image with respect to the position of a face within the feedback image as determined by the facial recognition unit, and an image capture initiator operable to initiate the capture of an output image subsequent to corruption of the feedback image.

### SUMMARY

According to an aspect of the invention, a method according to claim 1 and a device according to claim 7 is provided. Further aspects of the invention are defined by the dependent claims.

### Brief Description of Drawings

Fig. 1 shows an environment in which concepts described herein may be implemented.
Fig. 2 shows exemplary components included in a camera that may correspond to an imaging device that may be included in the environment of Fig. 1.
Fig. 3 shows exemplary components included in an augmented reality (AR) device that may correspond to the imaging device that may be included in the environment of Fig. 1.
Fig. 4 shows exemplary components included in a user device that may be included in the environment of Fig. 1.
Fig. 5 is a diagram illustrating exemplary components of a device included in the environment of Fig. 1.
Fig. 6 shows a flow diagram of an exemplary process to cause an imaging device to selectively capture one or more images in the environment of Fig. 1.
Fig. 7 shows a flow diagram of an exemplary process to cause an imaging device to selectively capture one or more images in the environment of Fig. 1.
Fig. 8 shows a flow diagram of an exemplary process to cause an imaging device to selectively capture one or more images in the environment of Fig. 1.
Fig. 9 shows a timeline that may be generated in the environment of Fig. 1.

### Description of Embodiments

The following detailed description refers to the accompanying drawings. The same reference numbers in different drawings may identify the same or similar elements.

The term "image," as used herein, may refer to a digital or an analog representation of visual information (e.g., a picture, a video, a photograph, an animation, etc). The term "camera," as used herein, may include a device that may capture and store images. For example, a digital camera may include an electronic device that may capture and store images electronically instead of using photographic film. A digital camera may be multifunctional, with some devices capable of recording sound and/or images. A "subject," as the term is used herein, is to be broadly interpreted to include any person, place, and/or thing capable of being captured as an image. The terms "user," "consumer," "subscriber," and/or "customer" may be used interchangeably. Also, the terms "user," "consumer," "subscriber," and/or "customer" are intended to be broadly interpreted to include a user device or a user of a user device. "Digital content," as referred to herein, includes one or more units of digital content that may be provided to a customer. The unit of digital content may include, for example, a segment of text, a defined set of graphics, a uniform resource locator (URL), a script, a program, an application or other unit of software, a media file (e.g., a movie, television content, music, etc.), a document, or an interconnected sequence of files (e.g., hypertext transfer protocol (HTTP) live streaming (HLS) media files).

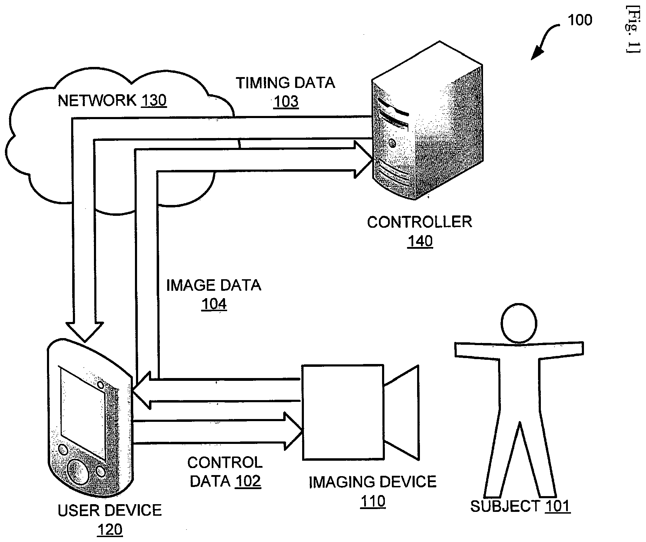

Fig. 1 shows an environment 100 in which concepts described herein may be implemented. As shown in Fig. 1, environment 100 may include an imaging device 110 to capture an image of a subject 101 based on control data 102 received from user device 120. Control data 102 may be generated by user device 120 based on timing data 103 received via network 130 from controller 140. Imaging device 110 may form image data 104 based on the captured image and may forward image data 104 to a user device 120 and/or via network 130 to controller 140.

Imaging device 110 may include a component to capture an image of subject 101. For example, imaging device 110 may include an optical sensor (not shown in Fig. 1) to detect optical attributes (e.g., a shade, a shape, a light intensity, etc.) associated with subject 101 (which correspond, for example, to a user associated with user device 120). Imaging device 110 may include a user interface (not shown in Fig. 1) that causes imaging device 110 to capture and/or alter image data 104. For example, imaging device 110 may include a button that, when selected by a user, causes imaging device 110 to generate image data 104 associated with subject 101. For example, imaging data 104 may include information identifying the optical attributes, and additional information identifying when and where the image was captured. Imaging device 110 may also include an interface (not shown in Fig. 1) to forward imaging data 104 to user device 120 and/or controller 140.

Imaging device 110 may be included as a component in or connected to user device 120. For example, imaging device 110 may correspond to a camera, a web camera, or other image capturing device coupled to user device 120.

User device 120 may include a mobile computing device, such as a smart phone, tablet computer, a mobile gaming device console, a laptop computer, or another type of hand-held computational or communication device. In another implementation, user device 120 may include a fixed device, such as a desktop computer or another computing device. In another implementation, user device 120 may correspond to a set-top box (STB) that receives digital content via one or more channels (e.g., Quadrature Amplitude Module (QAM) channels, Internet Protocol (IP) streams, etc.) for presentation to a user via display device.

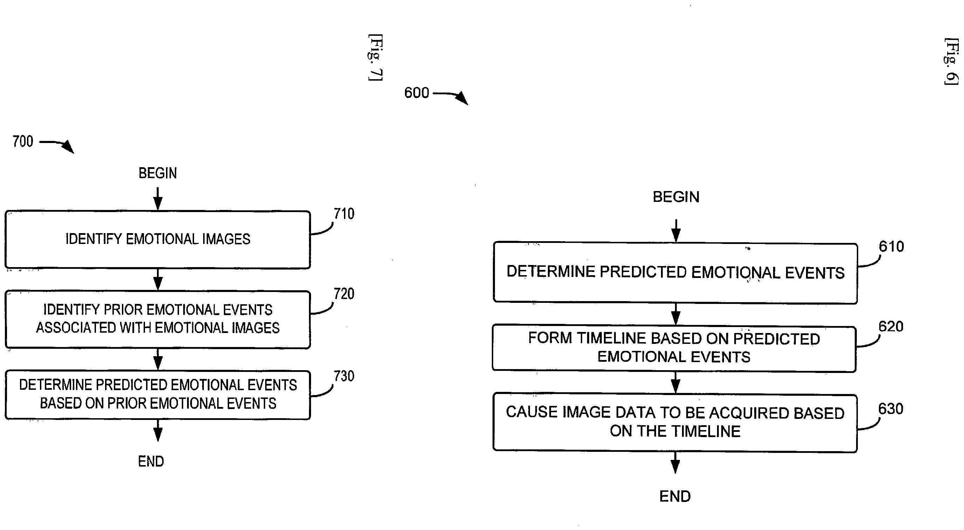

As described below with respect to Figs. 6-8, user device 120 may provide control data 102 directing imaging device 110 to automatically (e.g., without further user input) generate and/or transmit image data 104 at times associated with predicted emotional events. For example, timing data 103 from controller 140 may identify the times associated with predicted emotional events, and control data 102 from user device 120 may cause imaging device 110 to generate image data 104 based on the times associated with predicted emotional events.

Network 130 may include any network or combination of networks. In one implementation, network 130 may include one or more networks including, for example, a wireless public land mobile network (PLMN) (e.g., a Code Division Multiple Access (CDMA) 2000 PLMN, a Global System for Mobile Communications (GSM) PLMN, a Long Term Evolution (LTE) PLMN and/or other types of PLMNs), a telecommunications network (e.g., Public Switched Telephone Networks (PSTNs)), a local area network (LAN), a wide area network (WAN), a metropolitan area network (MAN), an intranet, the Internet, or a cable network (e.g., an optical cable network). Alternatively or in addition, network 130 may include a contents delivery network having multiple nodes that exchange data with user device 120. Although shown as a single element in Fig. 1, network 130 may include a number of separate networks that function to provide communications and/or services to user device 120.

In one implementation, network 130 may include a closed distribution network. The closed distribution network may include, for example, cable, optical fiber, satellite, or virtual private networks that restrict unauthorized alteration of contents delivered by a service provider. For example, network 130 may also include a network that distributes or makes available services, such as, for example, television services, mobile telephone services, and/or Internet services. Network 130 may be a satellite-based network and/ or a terrestrial-based network.

Controller 140 includes a device to determine predicted emotional events. As described below with respect to Figs. 6-8, controller 140 may determine, for example, when user device 120 will be near a person, place, and/or object likely to cause an emotional response by subject 101. In another implementation, controller 140 may determine, for example, particular portions of digital content (e.g., images, music, or text) to cause an emotional response by subject 101, and may cause imaging device 110 to capture and/or transmit an image of subject 101 when the particular portions of digital content are presented.

Although Fig. 1 depicts exemplary components of environment 100, in other implementations, environment 100 may include fewer components, additional components, different components, or differently arranged components than illustrated in Fig. 1. Furthermore, one or more components of environment 100 may perform one or more tasks described as being performed by one or more other components of environment 100. For example, controller 140 may be coupled to or included as a component of user device 120 such that user device 120 determines timing data 103. In other implementations, environment 100 could be realized locally in the imaging device 110 and/ or user device 120; or environment 100 may distributed in network 130, such as using a peer-to-peer connection. In another implementation, control device 140 may include a cloud server.

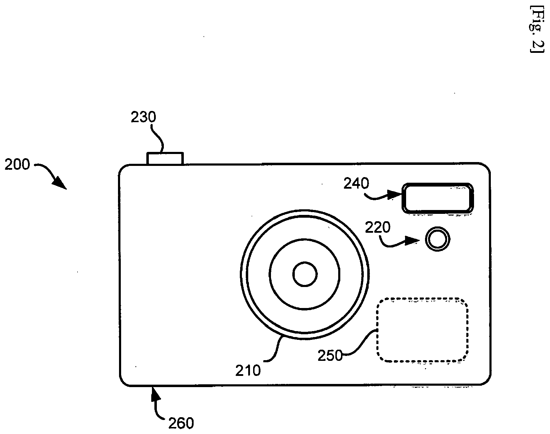

Fig. 2 shows exemplary components included in a camera 200 that may correspond to imaging device 110. Camera 200 may include different types of cameras, such as a point-and-shoot camera, single-lens reflex (SLR) camera (e.g., a camera in which images that a user sees in the viewfinder are obtained from the same light rays received for capturing images). As shown in Fig. 2, camera 200 may include, for example, a lens assembly 210, sensors 220, a button 230, a flash 240, a computing module 250, and a housing 260.

Lens assembly 210 may include a device for manipulating light rays from a given or a selected range, so that images in the range can be captured in a desired manner. Sensors 220 may collect and provide, to camera 200, information (e.g., acoustic, infrared, etc.) that is used to aid the user in capturing images. Button (or another user input) 230 may signal camera 200 to capture an image received by camera 200 via lens assembly 210 when the user presses (or otherwise activates) button 230. Flash 240 may include any type of flash unit used in cameras and may provide illumination for taking pictures. Computing module 250 may include one or more devices that provide computational capabilities of a computer. Fig. 2 shows computing module 250 in dotted lines, to indicate that computing module 250 is enclosed within housing 260. Housing 260 may provide a casing for components of camera 200 and may protect the components from outside elements.

During operation of camera 200, computing module 250 may receive input/signals from different components of camera 200 (e.g., sensors 220, touch screen, etc.), process the input/ signals, and/or control different components of camera 200. Computing module 250 may run applications, such as an image processing program, and interact with the user via input/ output components. As described with respect to Fig. 1, computing module 250 may receive control data 102 from user device 120 and may automatically (i.e., without a user selecting button 230) generate image data associated with subject 101.

Although Fig. 2 depicts exemplary components of camera 200, in other implementations, camera 200 may include fewer components, additional components, different components, or differently arranged components than illustrated in Fig. 2. Furthermore, one or more components of camera 200 may perform one or more tasks described as being performed by one or more other components of camera 200.

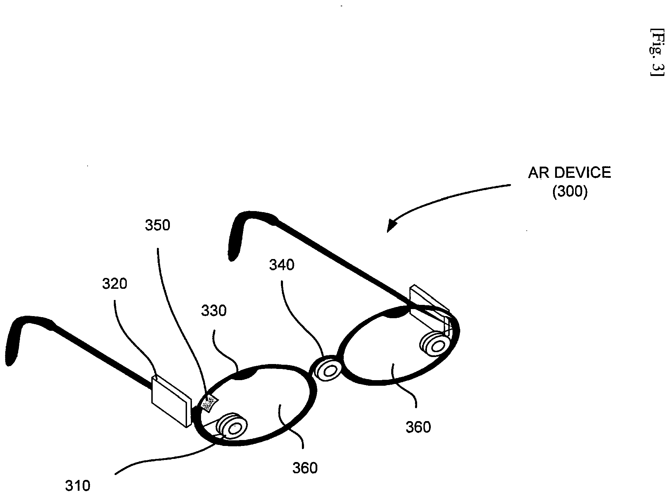

Fig. 3 shows exemplary components that may be included in an augmented reality (AR) device 300 that may correspond to imaging device 110 in one implementation. AR device 300 may correspond, for example, to a head-mounted display (HMD) that includes a display device paired to a headset, such as a harness or helmet. HMDs place images of both the physical world and virtual objects over the user's field of view. AR device 300 may also correspond to AR eyeglasses. For example, AR device 300 may include eye wear that employ cameras to intercept the real world view and re-display a augmented view through the eye pieces and devices in which the AR imagery is projected through or reflected off the surfaces of the eye wear lens pieces.

As shown in Fig. 3, AR device 300 may include, for example, a depth sensing camera 310, sensors 320, eye camera(s) 330, front camera 340, projector(s) 350, and lenses 360. Depth sensing camera 310 and sensors 320 may collect depth, position, and orientation information of objects viewed by a user in the physical world. For example, depth sensing camera 310 (also referred to as a "depth camera") may detect distances of objects relative to AR device 300. Sensors 320 may include any types of sensors used to provide information to AR device 300. Sensors 320 may include, for example, motion sensors (e.g., an accelerometer), rotation sensors (e.g., a gyroscope), and/or magnetic field sensors (e.g., a magnetometer).

Continuing with Fig. 3, eye cameras 330 may track eye movement to determine the direction in which the user is looking in the physical world. Front camera 340 may capture images (e.g., color/texture images) from surroundings, and projectors 350 may provide images and/or data to be viewed by the user in addition to the physical world viewed through lenses 360.

In operation, AR device 300 may receive control data 102 from user device 120 and may capture images (e.g., activate front camera 340) based on control data 102. For example, control data 102 may identify a time, a place, or a particular subject 101 such that front camera 340 is activated based on the identified time, place, or particular subject 101 (e.g., when the particular subject 101 is detected via depth sensing camera 310 and/or sensor 320).

Although Fig. 3 depicts exemplary components of AR device 300, in other implementations, AR device 300 may include fewer components, additional components, different components, or differently arranged components than illustrated in Fig. 3. Furthermore, one or more components of AR device 300 may perform one or more tasks described as being performed by one or more other components of AR device 300.

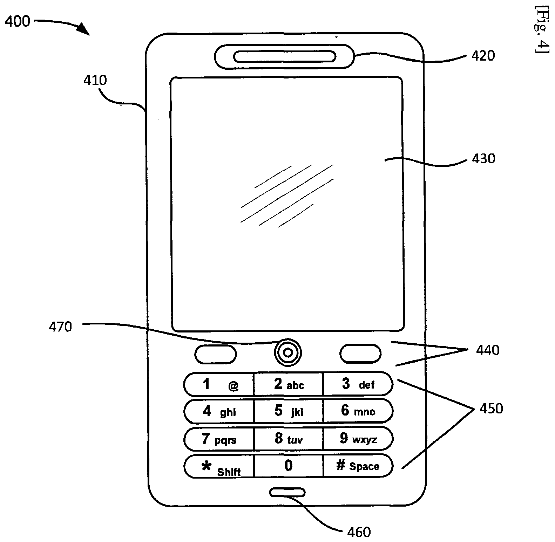

Fig. 4 is shows an exemplary device 400 that may correspond to user device 120. As shown in Fig. 4, user device 400 may include a housing 410, a speaker 420, a touch screen 430, control buttons 440, a keypad 450, a microphone 460, and/or a camera element 470. Housing 410 may include a chassis via which some or all of the components of user device 400 are mechanically secured and/or covered. Speaker 420 may include a component to receive input electrical signals from user device 400 and transmit audio output signals, which communicate audible information to a user of user device 400.

Touch screen 430 may include a component to receive input electrical signals and present a visual output in the form of text, images, videos and/or combinations of text, images, and/or videos which communicate visual information to the user of user device 400. In one implementation, touch screen 430 may display text input into user device 400, text, images, and/or video received from another device, and/or information regarding incoming or outgoing calls or text messages, emails, media, games, phone books, address books, the current time, etc.

Touch screen 430 may also include a component to permit data and control commands to be inputted into user device 400 via touch screen 430. For example, touch screen 430 may include a pressure sensor to detect a physical content of an input device to touch screen 430. Alternatively or in addition, a current and/or a field may be generated with respect to touch screen 430, and touch screen 430 may include a sensor to detect disruptions of the field and/or current associated with movements of the input device.

Control buttons 440 may include one or more buttons that accept, as input, mechanical pressure from the user (e.g., the user presses a control button or combinations of control buttons) and send electrical signals to a processor (not shown) that may cause user device 400 to perform one or more operations. For example, control buttons 440 may be used to cause user device 400 to transmit information. Keypad 450 may include a standard telephone keypad or another arrangement of keys.

Microphone 460 may include a component to receive audible information from the user and send, as output, an electrical signal that may be stored by user device 400, transmitted to another user device, or cause the device to perform one or more operations. Camera element 470 may be provided on a front or back side of user device 400, and may include a component to receive, as input, analog optical signals and send, as output, a digital image or video that can be, for example, viewed on touch screen 430, stored in the memory of user device 400, discarded and/or transmitted to another user device 400.

Although Fig. 4 depicts exemplary components of user device 400, in other implementations, user device 400 may include fewer components, additional components, different components, or differently arranged components than illustrated in Fig. 4. Furthermore, one or more components of user device 400 may perform one or more tasks described as being performed by one or more other components of user device 400.

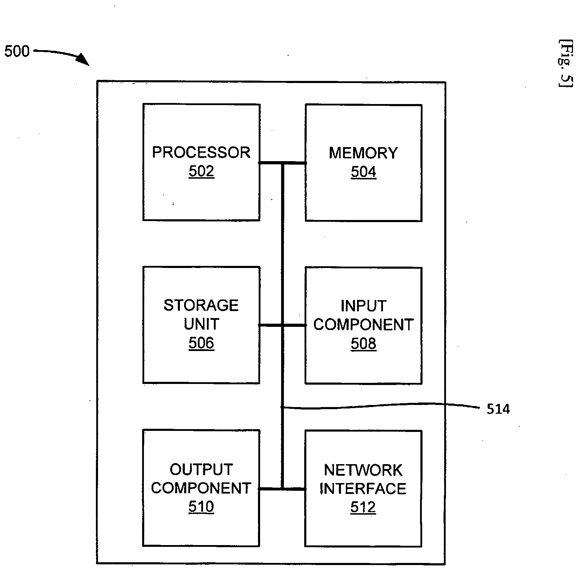

Fig. 5 is a diagram illustrating exemplary components of a device 500 which may represent a device included in environment 100 shown in Fig. 1. For example, device 500 may correspond to imaging device 110, user device 120, a component of network 130, and/or controller 140. As shown in Fig. 5, device 500 may include, for example, a processor 502, memory 504, storage unit 506, input component 508, output component 510, network interface 512, and communication path 514.

Processor 502 may include a processor, a microprocessor, an Application Specific Integrated Circuit (ASIC), a Field Programmable Gate Array (FPGA), and/or other processing logic (e.g., audio/video processor) capable of processing information and/or controlling device 500. Memory 504 may include static memory, such as read only memory (ROM), and/or dynamic memory, such as random access memory (RAM), or onboard cache, for storing data and machine-readable instructions. Storage unit 506 may include storage devices, such as a floppy disk, CD ROM, CD read/write (R/W) disc, and/or flash memory, as well as other types of storage devices.

Input component 508 and output component 510 may include a display screen, a keyboard, a mouse, a speaker, a microphone, a Digital Video Disk (DVD) writer, a DVD reader, Universal Serial Bus (USB) port, and/or other types of components for converting physical events or phenomena to and/or from digital signals that pertain to device 500.

Network interface 512 may include a transceiver that enables device 500 to communicate with other devices and/or systems. For example, network interface 512 may communicate via a network, such as the Internet, a terrestrial wireless network (e.g., a WLAN), a cellular network, a satellite-based network, a wireless personal area network (WPAN), etc. Additionally or alternatively, network interface 512 may include a modem, an Ethernet interface to a LAN, and/or an interface/connection for connecting device 500 to other devices (e.g., a Bluetooth interface, WiFi interface, etc.). For example, in one implementation, AR device 300 may communicate with user device 120 via Bluetooth interfaces.

Communication path 514 may provide an interface through which components of device 500 can communicate with one another.

In different implementations, device 500 may include additional, fewer, different, or differently arranged components than the ones illustrated in Fig. 5. For example, device 500 may include additional network interfaces, such as interfaces for receiving and sending data packets. In another example, device 500 may include a tactile input device.

Fig. 6 is a flow chart of an exemplary process 600 for causing image device 110 to selectively capture one or more images. In one exemplary implementation, process 600 may be performed by controller 140. In another exemplary implementation, some or all of process 600 may be performed by a device or collection of devices separate from, or in combination with controller 140, such as in combination with user device 120.

Process 600 may include determining predicted emotional events (block 610). A predicted event corresponds to a time when an emotional response is predicted from subject 101. A predicted emotional event may correspond to user device 120 approaching a particular place, object, and/or person of note. For example, a predicted event may correspond to user device 120 being proximate to both a particular place (e.g., a restaurant) and a particular person (e.g., a family member the user is meeting at a restaurant).

Controller 140 may cause an interface to be provided to a user (e.g., providing a graphical user interface, or GUI, via user device 120) to receive a user input identifying places, objects, and/or persons of note. In addition or alternatively, controller 140 device may identify the places, objects, and/or people of note based on data associated with user device 120. For example, contacts stored by user device 120 and/or communications exchanged with user device 120 may be reviewed to identify the people of note.

In one implementation, controller 140 may identify a presence of one of the identified places, objects, and/or people based on processing an image captured by imaging device 110. For example, controller 140 may store physical attributes associated with a particular place, object, or person and may determine whether the attribute (shape, shade, size, etc.) associated with the place, object, or person a subject corresponds to an attribute of a subject of a captured image.

Alternatively or in addition, controller 140 may determine that user device 120 is close to a particular place, object, or person when user device 120 receives a short range communication signal from a device associated with the particular place, object, or person. For example, user device 120 may establish a communication connection to the device associated with the particular place, object, or person using a short-range communications signaling protocol (e.g., WiFi or Bluetooth (registered trade mark)).

Controller 140 may determine locations associated with the places, objects, and/or people of note and may determine a location associated with user device 120 based on sensor reading from user device 120. In this implementation, a predicted emotional event may be associated with user device 120 is approaching a location associated with one of the identified places, objects, and/or people. A position of user device 120 may be determined, for example, based on global positioning system (GPS) information determined by user device 120. A location associated with user device 120 may also be determined based on processing signal strength (or other attribute) of signal received by user device 120 from one or more other devices, such as base stations (not shown).

In another implementation, a predicted emotional event may be associated with particular sensor data collected by user device 120. For example, a predicted emotional event may be associated with a change of more than a threshold amount in location, motion, light brightness levels, temperature, sound volume levels, etc. associated with user device 120. In addition or alternatively, a predicted emotional event may be associated with measurement data associated with a user of user device 120. For example, predicted emotional event may be associated with a change in body temperature, heart rate, breath rate, or other measurement data associated with the user.

In other implementations, emotional responses may be detected based on psycho-physical data, such as eye tracking, pupil dilation, or/or blink frequency; using a galvanic skin response (GSR), electrodermal response (EDR), psychogalvanic reflex (PGR), skin conductance response (SCR) or skin conductance level (SCL) to measure the electrical conductance of the skin of subject 101, which varies with its moisture level. Psycho-physical data may also be collected using medical imaging, such as using a Positron emission tomography (PET) nuclear medical imaging technique to produce a three-dimensional image or picture of functional processes in the body; using a Functional magnetic resonance imaging or functional MRI (fMRI) or other functional neuroimaging procedure to measure brain activity; using magnetic resonance spectroscopy (MRS), also known as nuclear magnetic resonance (NMR) spectroscopy, to identify metabolic changes in subject 101; using Single-photon emission computed tomography (SPECT, or less commonly, SPET) to image portions of user with gamma rays. In another implementation, emotional response may be detected based on skin color, heart rate, respiration rate, speech, etc. of subject 101 (or another person). In these examples, the psycho-physical data may be collected from subject 101 (or another person) to identify digital content that provokes an emotional change or a particular emotional state.

In another implementation, content analysis of surroundings and/or digital content presented to subject 101 (such as the posture or emotional states of people in the digital images) may be used to predict an emotional response. For example, an image of happy face may be expected to cause a happy response in subject 101.

Fig. 7 is a flow chart of an exemplary process 700 that corresponds to determining predicted emotional events in block 610. In one exemplary implementation, process 700 may be performed by controller 140. In another exemplary implementation, some or all of process 700 may be performed by a device or collection of devices separate from or in combination with controller 140.

Process 700 includes identifying emotional images associated with prior events (block 710). In block 710, controller 140 may process image data 104 captured by imaging device 110, or another device, to identify emotional expressions. For example, controller 140 may locate faces included in image data 104 and may process the position and movement of eyebrows, eyelids, cheeks, and/or mouth to identify an emotional state associated with a subject 101. For example, controller 140 may determine that a subject 101 is happy if image data 104 shows that the subject 101 is smiling.

For example, as described in an article entitled "Face Detection And Recognition Of Natural Human Emotion Using Markov Random Fields," by Ilias Maglogiannis, De-mosthenes Vouyioukas, Chris Aggelopoulos (Personal and Ubiquitous Computing, Volume 13, Issue 1, pp 95-101, January 1, 2009), skin detection may be performed in images using Markov random fields models for image segmentation and skin detection, eye and mouth detection and extraction may be performed using the hue, light, and value (HLV) color space of the specified eye and mouth region, and detecting the emotions pictured in the eyes and mouth, using edge detection and measuring the gradient of eyes' and mouth's region figure.

In one implementation, the image data 104 processed in block 710 may correspond to images of the user of user device 120. Thus, the identified emotional images may be specific to the particular user. In another implementation, the image data 104 processed in block 710 may be associated with images of multiple people.

Continuing with process 700 in Fig. 7, controller 140 identifies prior emotional events associated with the emotional images identified in process block 710 (block 720). Controller 140 may identify times and locations of imaging device 110 during the emotional images. For example, controller 140 may extract information from image data 104 identifying when and/or where the emotional images where captured by imaging device 110.

In another implementation, controller 140 may process image data 104 to identify a cause of a particular emotional event. For example, controller 140 may identify a person, place, or object associated with an emotional event. In one example, controller 140 may determine that a user is happy (e.g., smiling) when in the presence of a family member and/or at a particular location. In addition or alternatively, controller 140 may process a calendar or other information to determine an occasion associated with an emotional image. For example, controller 140 may determine that a user is generally happy (e.g., smiling) during a particular time (e.g., a national holiday) or in response to an event (e.g., a score at a sporting event).

Continuing with process 700 in Fig. 7, controller 140 determines predicted emotional events based on the identified prior emotional events (block 730). In block 730, controller 140 may process the prior emotional events to identify a time pattern associated the prior emotional events, and use the time pattern to determine the predicted emotional events. For example, controller 140 may use Markov chain analysis, time series, or other statistical techniques to determine trends and use these trends to predict future emotional events. For example, controller 140 may determine that certain emotional events occur periodically (e.g., a weekly sporting event), and may use this information to predict future emotional events.

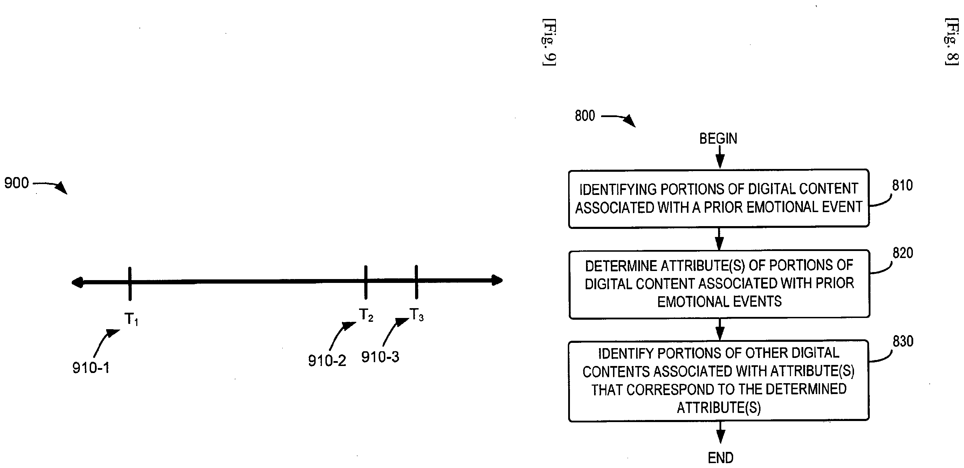

Fig. 8 is a flow chart of an exemplary process 800 to determine predicted emotional events in process block 730 based on the prior emotional events associated with the presentation of digital content. In one exemplary implementation, process 800 may be performed by controller 140. In another exemplary implementation, some or all of process 800 may be performed by a device or collection of devices separate from or in combination with controller 140.

Process 800 may include identifying portions of digital content associated with a prior emotional event (block 810). For example, controller 140 may determine a portion of the digital content presented to subject 101 at the times associated with the emotional events. For example, a prior emotional image (e.g., an image of subject 101 expressing an emotional response) may be associated with a scene from a movie, a written passage, or a portion of song.

Continuing with process 800, controller 140 may identify one or more attributes of a portion of digital content associated with a prior emotional event (block 820). For example, controller 140 may identify changes in light intensity, colors, and/or shapes associated with the portion of an image or movie and/or changes in sound attributes (such as volume) associated with a portion of music or a movie associated with a prior emotional event.

When the digital content corresponds to text, such as a book or article, controller 140 may identify words or phrases associated with the prior emotional event. For example, if imaging,device 110 corresponds to AR device 300, eye camera 330 may determine a location of a user's eyes during the emotional event, and controller 140 may use this information to identify portions of the text being read by the user during the emotional event. For example, as described in an article entitled "Identifying Expressions of Emotion in Text," by Saima Aman and Stan Szpakowicz (TSD'07 Proceedings of the 10th international conference on Text, Speech and Dialogue, Pages 196-205), an emotional response from text may be predicted by identifying emotional words in the text, evaluating the strength of emotional words (e.g., "love" or "adoration" indicating a stronger positive response that "like"), and the frequency that the emotional words appear in the text.

In one implementation, controller 140 may use image recognition techniques to identify a particular place or person being presented for display during an emotional event. Similarly, controller 140 may use speech-to-text techniques to determine words (e.g., dialog or lyrics) associated with a portion of a movie or a song presented to the user during an emotional event.

Continuing with Fig. 8, process 800 may further include identifying a portion of a digital content that include one or more attributes corresponding to the determined attributes (e.g., the attributes of portions of digital content associated with prior emotional events) (block 830). For example, controller 140 may identify portions a second digital content that depict a person, place, or object depicted in a portion of a first digital content associated with a prior emotion event. For example, if a prior emotional event is associated with images of a historical event, controller 140 may identify another portion of the digital content associated with the historical event.

Returning to Fig. 6, process 600 includes forming a timeline based on the predicted emotional events (block 620). For example, the timeline may correspond to a schedule when imaging device 110 may automatically (i.e., without additional user input) activate to capture and/or forward image data 104.

Fig. 9 shows an exemplary timeline 900 that may be generated by controller 140 in one implementation. Timeline 900 may include times 910. In the example shown in Fig. 9, timeline 900 includes three particular times T₁ (910-1), T₂ (910-2), and T₃ (910-3). A time 910 may correspond to a predicted emotional event (e.g., user device approaching a place, object, and/or person of note) determined in block 610. For example, a particular time 910 may correspond to a predicted arrival time of user device 120 at a particular location. This predicted arrival time may be determined based on dividing a distance between user device 120 and the particular location by a movement rate (i.e., velocity) associated with user device 120.

In another implementation, a time 910 may also be determined based on one more rules regarding timeline 900. A rule may specify, for example, a minimum and/or maximum number of images to capture/transmit during a time period; a minimum and/ or maximum duration of time between capturing/transmitting images, etc. A rule may also specify, for example, that images should not be captured during certain time periods (e.g., at night when the user is sleeping and/or desires privacy).

For example, if the number of predicted emotional events exceeds a threshold, a rule may cause controller 140 to disregard one or more of the predicted emotional events determined in block 610 when forming timeline 900. Similarly, if the number of predicted emotional events is less than a threshold, another rule may cause controller 140 to include one or more additional times 910, which are not associated with predicted emotional events, in timeline 900. In the example shown in Fig. 9, if controller 140 determines that a predicted emotional events associated with T₁ (910-1) and T₃ (910-3) are too far apart, such as when an insufficient number of predicted events are included the time duration between T₁ (910-1) and T₃ (910-3), controller 140 may include T₂ (910-2) in timeline 900.

In another implementation, a rule may cause controller 140 to change a time 910 associated with a predicted emotional event in timeline 900. Returning to the example shown in Fig. 9, if no predicted emotional events are associated with a time period with between T₁ (910-1) and T₃ (910-3), controller 140 may move (or reschedule) the predicted emotional event associated with T₃ (910-3) to T₂ (910-2).

Returning to Fig. 6, process 600 includes causing image data 104 to be acquired based on the timeline (block 630). For example, controller 140 may forward instructions to imaging device 110 to capture image data 104 at times 910 specified in timeline 900. For example, if imaging device 110 corresponds to AR device 300, controller 140 may forward instruction or otherwise cause front camera 340 to activate to capture image data 104.

In addition or alternatively, controller 140 may cause imaging device 110 to selectively connect to user device 120 and/or network 130 at the times 910 specified in timeline 900 to forward image data 104 only at those times 910. For example, if imaging device 110 corresponds to device 500, controller 140 may forward instruction to selectively activate network interface 512.

In another implementation, controller 140 may cause a network device connecting imaging device 110 to user device 120 and/or network 130, such as a router, to connect selectively to imaging device 110 at the times 910 included in timeline 900. In this implementation, controller 140 does not directly control the operation of imaging device 110 but may control a connection (i.e., via the network device) to imaging device 110 to control image data 104.

As described above with respect to process 800 in Fig. 8, portions of the digital content associated with predicted emotional events are may be identified in process block 830. In this implementation, process block 630 may include causing imaging device 110 to capture and/or transmit an image of subject 101 when one of identified portions of the digital content is presented to a user (e.g., when the portion of the digital content is rendered on user device 120). For example, imaging device 110 may capture an image of subject 101 when subject 101 is detected after a time 910. In another implementation, imaging device 110 may output an prompt (such as an audio noise) at a time 910 to indicate to a user that imaging device 110 should be pointed at a desired subject 101.

While a series of blocks has been described with regard to processes 600, 700, and 800 shown in Figs. 6-8, the order of the blocks may be modified in other implementations. Further, non-dependent blocks may be performed in parallel.

It will be apparent that systems and methods, as described above, may be implemented in many different forms of software, firmware, and hardware in the implementations illustrated in the figures. The actual software code or specialized control hardware used to implement these systems and methods is not limiting of the implementations. Thus, the operation and behavior of the systems and methods were described without reference to the specific software code--it being understood that software and control hardware can be designed to implement the systems and methods based on the description herein.

Further, certain portions, described above, may be implemented as a component or logic that performs one or more functions. A component or logic, as used herein, may include hardware, such as a processor, an ASIC, or a FPGA, or a combination of hardware and software (e.g., a processor executing software).

It should be emphasized that the terms "comprises" and "comprising," when used in this specification, are taken to specify the presence of stated features, integers, steps or components but do not preclude the presence or addition of one or more other features, integers, steps, components or groups thereof.

The scope of the invention is defined by the claims.

No element, act, or instruction used in the present application should be construed as critical or essential to the implementations unless explicitly described as such. Also, as used herein, the article "a" is intended to include one or more items. Where only one item is intended, the term "one" or similar language is used. Further, the phrase "based on" is intended to mean "based, at least in part, on" unless explicitly stated otherwise.

## Claims

1. A method, by a device, comprising:
determining (610), by a processor, predicted emotional events, wherein the predicted emotional events are associated with an emotional response by a subject; wherein determining (610) the predicted emotional events includes:
identifying (710) emotional images;
identifying (720) prior emotional events associated with the emotional images; and determining (730) the predicted emotional events based on the prior emotional events;
forming (620), by the processor, a timeline based on the predicted emotional events, wherein the timeline includes one or more times associated with the predicted emotional events; and
causing (630), by the processor, image data associated with the subject to be acquired based on the timeline by automatically activating an imaging device during one or more times associated with the predicted emotional events and capturing the image data associated with the subject with the imaging device during the one or more times associated with the predicted emotional events.

2. The method of claim 1, wherein determining (730) the predicted emotional events
based on the prior emotional events includes:
identifying times associated with the prior emotional events; and
determining the one or more times associated with the predicted emotional events based on the times associated with the prior emotional events.

3. The method of claim 1, wherein determining (730) the predicted emotional events
based on the prior emotional events includes:
identifying at least one of a person, place, or object associated with the emotional images; and determining the predicted emotional events based on the identified at least one of the person, the place, or the object.

4. The method of claim 1, wherein determining (730) the predicted emotional events based on the prior emotional events includes:
identifying (810) portions of digital content associated with a prior emotional event;
determining (820) an attribute of the portions of the digital content associated with prior emotional events;
identifying (830) another portion of the digital content that include one or more attributes corresponding to the determined attributes; and
determining the predicted emotional events based on the other portion of the digital content.

5. The method of claim 4, wherein the digital content is associated with an image, and wherein determining the attribute (820) includes:
performing image recognition on the image to identify at least one of a person, a place, or an object associated with one or more of the prior emotional events.

6. The method of claim 4, wherein the digital content is associated with text, and wherein determining the attribute (820) includes:
performing character recognition on the text to identify a word or a phrase associated with one or more of the prior emotional events.

7. A device (500) comprising:
a memory (504) configured to store information associated with prior emotional events, wherein the prior emotional events are associated with an emotional response by a subject; and
a processor (502) configured to:
determine predicted emotional events based on the prior emotional events, including:
identify emotional images associated with the subject, and
identify the prior emotional events associated with the emotional images,
form a timeline based on the predicted emotional events,
wherein the timeline includes one or more times associated with the predicted emotional events, and cause image data associated with the subject to be acquired based on the timeline.

8. The device of claim 7, wherein the processor (502), when determining the predicted emotional events, is further configured to:
identify times associated with the prior emotional events, and
determine the one or more times associated with the predicted emotional events based on the times associated with the prior emotional events.

9. The device of claim 7, wherein the processor (502), when determining the predicted emotional events, is further configured to:
identify at least one of a person, place, or object associated with the emotional images, and
determine the predicted emotional events based on the identified at least one of the person, the place, or the object.

10. The device of claim 7, wherein the processor (502), when determining the predicted emotional events, is further configured to:
identify portions of digital content associated with a prior emotional event,
determine an attribute of the portions of the digital content associated with prior emotional events,
identify another portions of the digital content associated with the attribute, and
determine the predicted emotional events based on the other portions of the digital content.

11. The device of claim 7, wherein the processor (502), when forming the timeline, is further configured to:
determine one or more additional times that are not associated with the predicted emotional events, wherein the processor determines the one or more additional times based on a rule regarding properties of the one or more times associated with the predicted emotional events, and
include, in the timeline, the one or more additional times.

12. The device of claim 7, wherein the processor (502), when causing the image data associated with the subject to be acquired based on the timeline, is further configured to at least one of: cause an imaging device (110) to capture the image data based on the one or more times included in the timeline, or
cause the imaging device (110) to transmit the image data based on the one or more times included in the timeline.

13. The device of claim 12, wherein the imaging device (110) includes at least one of:
a camera, or
an augmented reality device.

## Patentansprüche

1. Verfahren von einer Vorrichtung, umfassend:
Ermitteln (610) prognostizierter Gefühlsereignisse durch einen Prozessor, wobei die prognostizierten Gefühlsereignisse mit einer Gefühlsreaktion durch eine Person verbunden sind;
wobei das Ermitteln (610) der prognostizierten Gefühlsereignisse Folgendes umfasst:
Identifizieren (710) von Gefühlsbildern;
Identifizieren (720) früherer Gefühlsereignisse in Verbindung mit den Gefühlsbildern; und Ermitteln (730) der prognostizierten Gefühlsereignisse basierend auf den früheren Gefühlsereignissen;
Ausbilden (620) einer Zeitleiste durch den Prozessor basierend auf den prognostizierten Gefühlsereignissen, wobei die Zeitleiste eine oder mehrere Zeitpunkte in Verbindung mit den prognostizierten Gefühlsereignissen umfasst; und
Bewirken (630) durch den Prozessor, dass Bilddaten in Verbindung mit der Person basierend auf der Zeitleiste aufgenommen werden, indem automatisch eine bildgebende Vorrichtung während eines oder mehrerer Zeitpunkte in Verbindung mit den prognostizierten Gefühlsereignissen aktiviert wird und die Bilddaten in Verbindung mit der Person mit der bildgebenden Vorrichtung während des einen oder der mehreren Zeitpunkte in Verbindung mit den prognostizierten Gefühlsereignissen erfasst werden.

2. Verfahren nach Anspruch 1, wobei das Ermitteln (730) der prognostizierten Gefühlsereignisse basierend auf früheren Gefühlsereignissen Folgendes umfasst:
Identifizieren von Zeitpunkten in Verbindung mit früheren Gefühlsereignissen; und
Ermitteln des einen oder der mehreren Zeitpunkte in Verbindung mit den prognostizierten Gefühlsereignissen basierend auf den Zeitpunkten in Verbindung mit den früheren Gefühlsereignissen.

3. Verfahren nach Anspruch 1, wobei das Ermitteln (730) der prognostizierten Gefühlsereignisse basierend auf den früheren Gefühlsereignissen Folgendes umfasst:
Identifizieren mindestens eines aus einer Person, einem Ort oder einem Objekt in Verbindung mit den Gefühlsbildern; und Ermitteln der prognostizierten Gefühlsereignisse basierend auf dem identifizierten mindestens einen aus der Person, dem Ort oder dem Objekt.

4. Verfahren nach Anspruch 1, wobei das Ermitteln (730) der prognostizierten Gefühlsereignisse basierend auf den früheren Gefühlsereignissen Folgendes umfasst:
Identifizieren (810) von Teilen digitalen Inhalts in Verbindung mit einem früheren Gefühlsereignis;
Ermitteln (820) eines Attributs der Teile des digitalen Inhalts in Verbindung mit früheren Gefühlsereignissen;
Identifizieren (830) eines anderen Teils des digitalen Inhalts, das ein oder mehrere Attribute enthält, die den ermittelten Attributen entspricht/entsprechen; und
Ermitteln der prognostizierten Gefühlsereignisse basierend auf dem anderen Teil des digitalen Inhalts.

5. Verfahren nach Anspruch 4, wobei der digitale Inhalt mit einem Bild verbunden ist und wobei das Ermitteln des Attributs (820) Folgendes umfasst:
Durchführen der Bilderkennung an dem Bild, um mindestens eines aus einer Person, einem Ort oder einem Objekt in Verbindung mit einem oder mehreren der früheren Gefühlsereignisse zu identifizieren.

6. Verfahren nach Anspruch 4, wobei der digitale Inhalt mit einem Text verbunden ist und wobei das Ermitteln des Attributs (820) Folgendes umfasst:
Durchführen der Zeichenerkennung an dem Text, um ein Wort oder einen Ausdruck in Verbindung mit einem oder mehreren der früheren Gefühlsereignisse zu identifizieren.

7. Vorrichtung (500), umfassend:
einen Speicher (504), der konfiguriert ist, um Informationen in Verbindung mit früheren Gefühlsereignissen zu speichern, wobei die früheren Gefühlsereignisse mit einer Gefühlsreaktion durch eine Person verbunden sind; und
einen Prozessor (502), der konfiguriert ist, um:
prognostizierte Gefühlsereignisse basierend auf den früheren Gefühlsereignissen zu ermitteln, umfassend:
Gefühlsbilder in Verbindung mit der Person zu identifizieren und die früheren Gefühlsereignisse in Verbindung mit den Gefühlsbildern zu identifizieren,
eine Zeitleiste basierend auf den prognostizierten Gefühlsereignissen zu bilden,
wobei die Zeitleiste einen oder mehrere Zeitpunkte in Verbindung mit den prognostizierten Gefühlsereignissen umfasst, und zu bewirken, dass Bilddaten in Verbindung mit der Person basierend auf der Zeitleiste aufgenommen werden.

8. Vorrichtung nach Anspruch 7, wobei der Prozessor (502) beim Ermitteln der prognostizierten Gefühlsereignisse ferner konfiguriert ist, um:
Zeitpunkte in Verbindung mit den früheren Gefühlsereignissen zu identifizieren und
den einen oder die mehreren Zeitpunkte in Verbindung mit den prognostizierten Gefühlsereignissen basierend auf den Zeitpunkten in Verbindung mit den früheren Gefühlsereignissen zu ermitteln.

9. Vorrichtung nach Anspruch 7, wobei der Prozessor (502) beim Ermitteln der prognostizierten Gefühlsereignisse ferner konfiguriert ist, um:
mindestens eines aus einer Person, einem Ort oder einem Objekt in Verbindung mit den Gefühlsbildern zu identifizieren und die prognostizierten Gefühlsereignisse basierend auf dem identifizierten mindestens einen aus der Person, dem Ort oder dem Objekt zu ermitteln.

10. Vorrichtung nach Anspruch 7, wobei der Prozessor (502) beim Ermitteln der prognostizierten Gefühlsereignisse ferner konfiguriert ist, um:
Teile digitalen Inhalts in Verbindung mit einem früheren Gefühlsereignis zu identifizieren,
ein Attribut der Teile des digitalen Inhalts in Verbindung mit früheren Gefühlsereignissen zu ermitteln,
andere Teile des digitalen Inhalts in Verbindung mit dem Attribut zu identifizieren und die prognostizierten Gefühlsereignisse basierend auf den anderen Teilen des digitalen Inhalts zu ermitteln.

11. Vorrichtung nach Anspruch 7, wobei der Prozessor (502) beim Ausbilden der Zeitleiste ferner konfiguriert ist, um:
einen oder mehrere zusätzliche Zeitpunkte zu ermitteln, die nicht mit den prognostizierten Gefühlsereignissen verbunden sind, wobei der Prozessor den einen oder die mehreren zusätzlichen Zeitpunkte basierend auf einer Regel hinsichtlich der Eigenschaften des einen oder der mehreren Zeitpunkte in Verbindung mit den prognostizierten Gefühlsereignissen ermittelt, und
in die Zeitleiste den einen oder die mehreren zusätzlichen Zeitpunkte aufzunehmen.

12. Vorrichtung nach Anspruch 7, wobei der Prozessor (502), wenn er bewirkt, dass die Bilddaten in Verbindung mit der Person basierend auf der Zeitleiste aufgenommen werden, ferner konfiguriert ist, um mindestens eines der Folgenden auszuführen:
zu bewirken, dass eine bildgebende Vorrichtung (110) die Bilddaten basierend auf dem einen oder den mehreren Zeitpunkten, die in die Zeitleiste aufgenommen sind, erfasst, oder
zu bewirken, dass die bildgebende Vorrichtung (110) die Bilddaten basierend auf dem einen oder den mehreren Zeitpunkten, die in die Zeitleiste aufgenommen sind, übermittelt.

13. Vorrichtung nach Anspruch 12, wobei die bildgebende Vorrichtung (110) mindestens eines der Folgenden umfasst:
eine Kamera oder
eine Vorrichtung für erweiterte Realität.

## Revendications

1. Procédé, par un dispositif, comprenant :
la détermination (610), par un processeur, d'événements émotionnels prédits, dans lequel les événements émotionnels prédits sont associés à une réponse émotionnelle d'un sujet ; dans lequel la détermination (610) des événements émotionnels prédits inclut :
l'identification (710) d'images émotionnelles ;
l'identification (720) d'événements émotionnels antérieurs associés aux images émotionnelles ; et
la détermination (730) des événements émotionnels prédits sur la base des événements émotionnels antérieurs ;
la formation (620), par le processeur, d'une chronologie basée sur les événements émotionnels prédits, dans lequel la chronologie inclut un ou plusieurs moments associés aux événements émotionnels prédits ; et
le fait d'amener (630), par le processeur, l'acquisition de données d'image associées au sujet sur la base de la chronologie en activant automatiquement un dispositif d'imagerie pendant un ou plusieurs moments associés aux événements émotionnels prédits et en capturant les données d'image associées au sujet avec le dispositif d'imagerie pendant les un ou plusieurs moments associés aux événements émotionnels prédits.

2. Procédé selon la revendication 1, dans lequel la détermination (730) des événements émotionnels prédits sur la base des événements émotionnels antérieurs inclut :
l'identification de moments associés aux événements émotionnels antérieurs ; et
la détermination des un ou plusieurs moments associés aux événements émotionnels prédits sur la base des moments associés aux événements émotionnels antérieurs.

3. Procédé selon la revendication 1, dans lequel la détermination (730) des événements émotionnels prédits sur la base des événements émotionnels antérieurs inclut :
l'identification d'au moins un élément parmi une personne, un lieu ou un objet associé aux images émotionnelles ; et
la détermination des événements émotionnels prédits sur la base de l'au moins un élément identifié parmi la personne, le lieu ou l'objet.

4. Procédé selon la revendication 1, dans lequel la détermination (730) des événements émotionnels prédits sur la base des événements émotionnels antérieurs inclut :
l'identification (810) de parties de contenu numérique associées à un événement émotionnel antérieur ;
la détermination (820) d'un attribut des parties du contenu numérique associées aux événements émotionnels antérieurs ;
l'identification (830) d'une autre partie du contenu numérique qui inclut un ou plusieurs attributs correspondant aux attributs déterminés ; et
la détermination des événements émotionnels prédits sur la base de l'autre partie du contenu numérique.

5. Procédé selon la revendication 4, dans lequel le contenu numérique est associé à une image, et dans lequel la détermination de l'attribut (820) inclut :
la réalisation d'une reconnaissance d'image sur l'image pour identifier au moins un élément parmi une personne, un lieu ou un objet associé à un ou plusieurs des événements émotionnels antérieurs.

6. Procédé selon la revendication 4, dans lequel le contenu numérique est associé à un texte, et dans lequel la détermination de l'attribut (820) inclut :
la réalisation d'une reconnaissance de caractères sur le texte pour identifier un mot ou une phrase associés à un ou plusieurs des événements émotionnels antérieurs.

7. Dispositif (500) comprenant :
une mémoire (504) conçue pour stocker des informations associées à des événements émotionnels antérieurs, dans lequel les événements émotionnels antérieurs sont associés à une réponse émotionnelle d'un sujet ; et
un processeur (502) conçu pour :
déterminer des événements émotionnels prédits sur la base des événements émotionnels antérieurs, incluant :
l'identification d'images émotionnelles associées au sujet, et
l'identification des événements émotionnels antérieurs associés aux images émotionnelles,
la formation d'une chronologie sur la base des événements émotionnels prédits,
dans lequel la chronologie inclut un ou plusieurs moments associés aux événements émotionnels prédits, et le fait d'amener l'acquisition de données d'image associées au sujet sur la base de la chronologie.

8. Dispositif selon la revendication 7, dans lequel le processeur (502), lors de la détermination des événements émotionnels prédits, est en outre conçu pour :
identifier des moments associés aux événements émotionnels antérieurs ; et
déterminer les un ou plusieurs moments associés aux événements émotionnels prédits sur la base des moments associés aux événements émotionnels antérieurs.

9. Dispositif selon la revendication 7, dans lequel le processeur (502), lors de la détermination des événements émotionnels prédits, est en outre conçu pour :
identifier au moins un élément parmi une personne, un lieu ou un objet associé aux images émotionnelles ; et
déterminer les événements émotionnels prédits sur la base de l'au moins un élément identifié parmi la personne, le lieu ou l'objet.

10. Dispositif selon la revendication 7, dans lequel le processeur (502), lors de la détermination des événements émotionnels prédits, est en outre conçu pour :
identifier des parties de contenu numérique associées à un événement émotionnel antérieur,
déterminer un attribut des parties du contenu numérique associées à des événements émotionnels antérieurs,
identifier d'autres parties du contenu numérique associées à l'attribut, et
déterminer les événements émotionnels prédits sur la base des autres parties du contenu numérique.

11. Dispositif selon la revendication 7, dans lequel le processeur (502), lors de la formation de la chronologie, est en outre conçu pour :
déterminer un ou plusieurs moments supplémentaires qui ne sont pas associés aux événements émotionnels prédits, dans lequel le processeur détermine les un ou plusieurs moments supplémentaires sur la base d'une règle concernant des propriétés des un ou plusieurs moments associés aux événements émotionnels prédits, et
inclure, dans la chronologie, les un ou plusieurs moments supplémentaires.

12. Dispositif selon la revendication 7, dans lequel le processeur (502), lorsqu'il entraîne l'acquisition des données d'image associées au sujet sur la base de la chronologie, est en outre conçu pour au moins l'une des opérations suivantes :
le fait d'amener un dispositif d'imagerie (110) à capturer les données d'image sur la base des un ou plusieurs moments inclus dans la chronologie, ou
le fait d'amener un dispositif d'imagerie (110) à transmettre les données d'image sur la base des un ou plusieurs moments inclus dans la chronologie.

13. Dispositif selon la revendication 12, dans lequel le dispositif d'imagerie (110) inclut au moins l'un des éléments suivants :
une caméra, ou
un dispositif de réalité augmentée.
